# FASCICULE DE BREVET EUROPEEN

(11) **EP 2 627 772 B1**
(45) Date de publication et mention de la délivrance du brevet: **21.12.2016**
(21) Numéro de dépôt: 11776909.1
(22) Date de dépôt: 14.10.2011
(51) Int. Cl.: C12N 15/82, C12N 9/16

(54) **OBTENTION DE PLANTES AYANT UNE TOLÉRANCE AMÉLIORÉE À UN DÉFICIT HYDRIQUE**
HERSTELLUNG VON PFLANZEN MIT VERBESSERTER WASSERMANGELTOLERANZ
PRODUCTION OF PLANTS WITH IMPROVED TOLERANCE TO WATER DEFICIT

(30) Priorité: 15.10.2010 FR 1004058
(43) Date de publication de la demande: 21.08.2013
(73) Titulaire: Genoplante-Valor, 75015 Paris (FR)
(72) Inventeur: ROUSTER, Jacques, F-63730 Mirefleurs (FR); SALLAUD, Christophe, F-63110 Beaumont (FR); COURSOL, Sylvie, F-75012 Paris (FR); ZIVY, Michel, F-75010 Paris (FR); VIRLOUVET, Laetitia, F-91360 Épinay-sur-Orge (FR); WELCKER, Claude, F-34980 Montferrier-sur-Lez (FR)
(74) Mandataire: Gevers & Orès
(86) Numéro de dépôt international: PCT/IB2011/054568
(87) Numéro de publication internationale: WO 2012/049661

(56) Documents cités:
- WO-A2-01/38502
- WO-A2-2007/049275
- US-A1- 2009 094 717

<110> GENOPLANTE-VALOR ROUSTER, Jacques SALLAUD, Christophe COURSOL, Sylvie ZIVY, Michel VIRLOUVET, Laetitia WELCKER, Claude
<120> OBTENTION DE PLANTES AYANT UNE TOLERANCE AMELIOREE A UN DEFICIT HYDRIQUE
<130> MJP/XRN/cc-F1516/39/PCT
<150> FR 1004058
   <151> 2010-10-15
<160> 9
<170> PatentIn version 3.5
<210> 1
   <211> 363
   <212> PRT
   <213> Zea mays
<400> 1
   Met Leu Arg Cys His Ala Pro Pro Gln Cys Gly Arg Ala Pro Leu Arg
   1               5                   10                  15
   His His Gly Arg Trp Glu Ser Ser Pro Ala Pro Gly Val Val Val Arg
               20                  25                  30
   Cys Thr Arg Gly Ala Pro Gln Val Ser Gly Ile Glu Ala Ala Ser Pro
           35                  40                  45
   Asp His Ala Ala Ala Thr Ala Val Ala Ala Lys Ala Glu Gly Gly Asp
       50                  55                  60
   Ala Arg Pro Ser Leu Ala Glu Arg Leu Arg Leu Gly Ser Leu Leu Glu
   65                  70                  75                  80
   Asp Gly Leu Ser Tyr Lys Glu Ser Phe Ile Val Arg Cys Tyr Glu Val
                   85                  90                  95
   Gly Ile Asn Lys Thr Ala Thr Val Glu Thr Ile Ala Asn Leu Leu Gln
               100                 105                 110
   Glu Val Gly Cys Asn His Ala Gln Ser Val Gly Phe Ser Thr Asp Gly
           115                 120                 125
   Phe Ala Thr Thr Thr Thr Met Arg Lys Leu Gly Leu Ile Trp Val Thr
       130                 135                 140
   Asn Arg Met His Ile Glu Ile Tyr Lys Tyr Pro Ala Trp Gly Asp Val
   145                 150                 155                 160
   Val Glu Ile Glu Thr Trp Cys Gln Glu Asp Gly Lys Ile Gly Thr Arg
                   165                 170                 175
   Arg Asp Trp Ile Leu Lys Asp Leu Cys Thr Gly Glu Val Thr Gly Arg
               180                 185                 190
   Ala Thr Ser Lys Trp Val Met Met Asn Gln Asn Thr Arg Arg Leu Gln
           195                 200                 205
   Arg Val Ser Asp Asp Val Arg Asp Glu Val Phe Ile His Cys Pro Lys
       210                 215                 220
   Thr Pro Arg Leu Ala Phe Pro Glu Glu Asn Asn Gly Ser Leu Lys Lys
   225                 230                 235                 240
   Ile Pro Asn Leu Ser Asp Pro Ala Gln Tyr Ser Arg Leu Gly Leu Val
                   245                 250                 255
   Pro Arg Arg Ala Asp Leu Asp Met Asn Gln His Val Asn Asn Val Thr
               260                 265                 270
   Tyr Ile Gly Trp Val Leu Glu Ser Ile Pro Gln Asp Ile Ile Asp Thr
           275                 280                 285
   His Glu Leu Gln Thr Ile Thr Leu Asp Tyr Arg Arg Glu Cys Gln His
       290                 295                 300
   Asp Asp Ile Val Asp Ser Leu Thr Tyr Val Glu Glu Gly Glu Glu Arg
   305                 310                 315                 320
   Ser Met Asn Gly Ser Ala Ser Ser Val Pro His Thr Glu Gln Arg Arg
                   325                 330                 335
   Gln Phe Leu His Cys Leu Arg Phe Ala Ala Asn Gly Asp Glu Ile Asn
               340                 345                 350
   Arg Gly Arg Thr Val Trp Arg Lys Leu Ala Arg
           355                 360
<210> 2
   <211> 353
   <212> PRT
   <213> Brachypodium distachyon
<400> 2
   Met Leu Arg Cys His Thr Pro Pro Gln Cys Arg Pro Ala Pro Leu Gly
   1               5                   10                  15
   Arg Arg Gly Leu Pro Arg Ala Ala Ala Glu Val Val Arg Cys Ala Ala
               20                  25                  30
   Arg Gly Ser Arg Arg Leu Pro Ser Leu Ile Val Ala Ser Ser Ser Ser
           35                  40                  45
   Ala Glu Ala Ala Val Thr Cys Gly Glu Ser Leu Ala Glu Arg Leu Arg
       50                  55                  60
   Met Gly Ser Leu Leu Glu Asp Gly Leu Ser Tyr Lys Glu Ser Phe Ile
   65                  70                  75                  80
   Val Arg Cys Tyr Glu Val Gly Ile Asn Lys Thr Ala Thr Val Glu Thr
                   85                  90                  95
   Ile Ala Asn Leu Leu Gln Glu Val Gly Cys Asn His Ala Gln Ser Val
               100                 105                 110
   Gly Phe Ser Thr Asp Gly Phe Ala Thr Thr Thr Thr Met Arg Glu Leu
           115                 120                 125
   Gly Leu Ile Trp Val Thr Asn Arg Met His Ile Glu Ile Tyr Lys Tyr
       130                 135                 140
   Pro Ala Trp Gly Asp Val Val Glu Ile Glu Thr Trp Cys Gln Ala Asp
   145                 150                 155                 160
   Gly Arg Ile Gly Thr Arg Arg Asp Trp Ile Ile Lys Asp Leu Ala Ser
                   165                 170                 175
   Gly Glu Val Ile Gly Arg Ala Thr Ser Lys Trp Leu Met Met Asn Gln
               180                 185                 190
   Ser Thr Arg Arg Leu Gln Arg Val Ser Asp Glu Val Arg Asp Glu Val
           195                 200                 205
   Phe Val His Cys Pro Lys Thr Pro Arg Leu Ala Phe Pro Glu Glu Asn
       210                 215                 220
   Asn Gly Ser Leu Lys Asn Ile Pro Ile Leu Thr Asp Pro Ala Gln Tyr
   225                 230                 235                 240
   Ser Arg Leu Gly Leu Val Pro Arg Arg Ala Asp Leu Asp Met Asn Gln
   245 250 255
   His Val Asn Asn Val Thr Tyr Ile Gly Trp Val Leu Glu Ser Ile Pro
   260 265 270
   Gln Asp Ile Ile Asp Thr His Glu Leu Gln Thr Ile Thr Leu Asp Tyr
   275 280 285
   Arg Arg Glu Cys Gln His Asp Asp Ile Val Asp Ser Leu Thr Tyr Val
   290 295 300
   Glu Glu Gly Glu Ala Lys Ser Ser Asn Gly Ser Ala Phe Ala Ala Pro
   305 310 315 320
   His Pro Gln Glu Gln Cys Gln Phe Leu His Cys Leu Arg Phe Ala Gly
   325 330 335
   Gly Gly Gly Glu Leu Asn Arg Gly Arg Thr Val Trp Arg Lys Leu Ser
   340 345 350
   Arg
<210> 3
   <211> 358
   <212> PRT
   <213> Triticum aestivum
<400> 3
   Met Leu Arg Cys His Ile Pro Pro Gln Cys Gly Pro Ala Pro Leu Gly
   1               5                   10                  15
   Arg Arg Gly Leu Pro Arg Ala Val Arg Cys Ala Ala Arg Ala Pro Ser
               20                  25                  30
   Arg Gly Ala Val Ala Ala Ala Ala Ala Ala Ala Ala Gln Val Ala Ala
           35                  40                  45
   Ala Ala Val Ala Thr Ala Glu Gly Arg Glu Gly Ala Glu Arg Pro Gly
       50                  55                  60
   Leu Ala Glu Arg Leu Arg Met Gly Ser Leu Leu Glu Asp Gly Leu Ser
   65                  70                  75                  80
   Tyr Lys Glu Ser Phe Ile Val Arg Cys Tyr Glu Val Gly Ile Asn Lys
                   85                  90                  95
   Thr Ala Thr Val Glu Thr Ile Ala Asn Leu Leu Gln Glu Val Gly Cys
   100 105 110
   Asn His Ala Gln Ser Val Gly Phe Ser Thr Asp Gly Phe Ala Thr Thr
   115 120 125
   Thr Thr Met Arg Glu Leu Gly Leu Ile Trp Val Thr Asn Arg Met His
   130 135 140
   Ile Glu Ile Tyr Lys Tyr Pro Ala Trp Gly Asp Val Val Glu Ile Glu
   145 150 155 160
   Thr Trp Cys Gln Ala Asp Gly Lys Ile Gly Thr Arg Arg Asp Trp Ile
   165 170 175
   Leu Lys Asp Leu Ala Asn Gly Glu Val Ile Gly Arg Ala Thr Ser Lys
   180 185 190
   Trp Val Met Met Asn Gln Asn Thr Arg Arg Leu Gln Arg Val Ser Asp
   195 200 205
   Glu Val Arg Asp Glu Val Phe Ile His Cys Pro Lys Ser Pro Arg Leu
   210 215 220
   Ala Phe Pro Glu Glu Asn Asn Gly Ser Leu Lys Lys Ile Pro Val Leu
   225 230 235 240
   Thr Asp Pro Ala Gln His Ser Arg Leu Gly Leu Val Pro Arg Arg Ala
   245 250 255
   Asp Leu Asp Met Asn Gln His Val Asn Asn Val Thr Tyr Ile Gly Trp
   260 265 270
   Val Leu Glu Ser Ile Pro Gln Asp Ile Ile Asp Thr His Glu Leu Gln
   275 280 285
   Thr Ile Thr Leu Asp Tyr Arg Arg Glu Cys Gln His Asp Asp Ile Val
   290 295 300
   Asp Ser Leu Thr Tyr Ile Glu Gly Glu Glu Ile Asn Ser Asn Gly Ser
   305 310 315 320
   Leu Phe Ser Ala Pro His Pro Glu Glu Gln Arg Gln Phe Leu His Cys
   325 330 335
   Leu Arg Phe Ala Gly Ala Gly Asp Glu Ile Asn Arg Gly Arg Thr Val
   340 345 350
   Trp Arg Lys Leu Ala Arg
   355
<210> 4
   <211> 356
   <212> PRT
   <213> Oryza sativa
<400> 4
   Met Leu Arg Cys His Thr Pro Pro Gln Cys Arg Leu Gly Ala Gly Gly
   1               5                   10                  15
   Ala Gly Ala Gly Val Leu Leu Arg Gln Arg Ser Glu Val Ala Val Arg
               20                  25                  30
   Cys Arg Ala Gln Gln Val Ser Gly Val Glu Ala Ala Ala Gly Thr Pro
           35                  40                  45
   Ala Ala Arg Ala Ala Val Glu Gly Gly Glu Arg Thr Ser Leu Ala Glu
       50                  55                  60
   Arg Leu Arg Leu Gly Ser Leu Leu Glu Asp Gly Leu Ser Tyr Lys Glu
   65                  70                  75                  80
   Ser Phe Ile Val Arg Cys Tyr Glu Val Gly Ile Asn Lys Thr Ala Thr
                   85                  90                  95
   Val Glu Thr Ile Ala Asn Leu Leu Gln Glu Val Gly Cys Asn His Ala
               100                 105                 110
   Gln Ser Val Gly Phe Ser Thr Asp Gly Phe Ala Thr Thr Thr Thr Met
           115                 120                 125
   Arg Lys Leu Gly Leu Ile Trp Val Thr Asn Arg Met His Ile Glu Ile
       130                 135                 140
   Tyr Lys Tyr Pro Ala Trp Gly Asp Val Val Glu Ile Glu Thr Trp Cys
   145                 150                 155                 160
   Gln Glu Asp Gly Lys Ile Gly Thr Arg Arg Asp Trp Ile Leu Lys Asp
                   165                 170                 175
   Leu Ala Asn Gly Glu Val Ile Gly Arg Ala Thr Ser Lys Trp Val Met
               180                 185                 190
   Met Asn Gln Asn Thr Arg Arg Leu Gln Arg Val Ser Asp Asp Val Arg
           195                 200                 205
   Asp Glu Val Phe Val His Cys Pro Lys Thr Pro Arg Leu Ala Phe Pro
       210                 215                 220
   Glu Glu Asn Asn Gly Ser Leu Lys Lys Ile Pro Val Leu Thr Asp Pro
   225                 230                 235                 240
   Ala Gln His Ser Arg Leu Gly Leu Val Pro Arg Arg Ala Asp Leu Asp
                   245                 250                 255
   Met Asn Gln His Val Asn Asn Val Thr Tyr Ile Gly Trp Val Leu Glu
               260                 265                 270
   Ser Ile Pro Gln Asp Ile Ile Asp Thr His Glu Leu Gln Thr Ile Thr
           275                 280                 285
   Leu Asp Tyr Arg Arg Glu Cys Gln His Asp Asp Ile Val Asp Ser Leu
       290                 295                 300
   Thr Tyr Ile Glu Glu Gly Glu Glu Lys Ser Ser Asn Gly Ser Ala Phe
   305                 310                 315                 320
   Ala Ala Pro His Pro Glu Glu Gln Arg Gln Phe Leu His Cys Leu Arg
                   325                 330                 335
   Phe Ala Gly Asn Gly Asn Glu Ile Asn Arg Gly Arg Thr Val Trp Arg
               340                 345                 350
   Lys Leu Ala Arg
           355
<210> 5
   <211> 366
   <212> PRT
   <213> Sorghum bicolor
<400> 5
   Met Leu Arg Cys Pro Thr Gln Pro Gln Cys Gly Arg Ala Pro Leu Arg
   1               5                   10                  15
   His His Gly Arg Arg Glu Ser Pro Pro Ser Ala Ala Pro Gly Val Val
               20                  25                  30
   Val Arg Cys Ala Arg Gly Ala Pro Gln Val Ser Arg Ile Glu Ala Ala
           35                  40                  45
   Ser Pro Val Ala Ala Thr Thr Ala Ala Ala Ala Ala Lys Ala Glu Arg
       50                  55                  60
   Gly Asp Ala Arg Pro Ser Leu Ala Glu Arg Leu Arg Leu Gly Ser Leu
   65                  70                  75                  80
   Leu Glu Asp Gly Leu Ser Tyr Lys Glu Ile Phe Ile Val Arg Ser Tyr
                   85                  90                  95
   Glu Val Gly Ile Asn Lys Thr Ala Thr Val Glu Thr Ile Ala Asn Leu
               100                 105                 110
   Leu Gln Glu Val Gly Cys Ser His Ala Gln Ser Leu Gly Phe Ser Thr
           115                 120                 125
   Asp Gly Phe Ala Thr Thr Thr Ser Met Arg Lys Leu Gly Leu Ile Trp
       130                 135                 140
   Val Thr Asn Arg Met His Ile Glu Ile Tyr Lys Tyr Pro Ala Trp Gly
   145                 150                 155                 160
   Asp Val Val Glu Ile Glu Thr Trp Cys Gln Ala Asp Gly Arg Met Gly
                   165                 170                 175
   Thr Arg Arg Asp Trp Ile Leu Lys Asp Leu Ala Asn Gly Glu Val Ile
               180                 185                 190
   Gly Arg Ala Thr Ser Lys Trp Val Thr Met Asn Gln Asn Thr Arg Arg
           195                 200                 205
   Leu Gln Arg Val Ser Asp Glu Val Arg Asp Glu Val Phe Ile His Cys
       210                 215                 220
   Pro Lys Thr Pro Arg Leu Ala Phe Pro Glu Glu Asn Asn Gly Ser Leu
   225                 230                 235                 240
   Lys Lys Ile Pro Asn Leu Ser Asp Ser Ser Gln Tyr Ser Arg Leu Gly
                   245                 250                 255
   Leu Val Pro Arg Arg Ala Asp Leu Asp Met Asn Gln His Val Asn Asn
               260                 265                 270
   Val Thr Tyr Ile Gly Trp Val Leu Glu Ser Ile Pro Gln Asp Ile Ile
           275                 280                 285
   Asp Thr His Glu Leu Gln Thr Ile Thr Leu Asp Tyr Arg Arg Glu Cys
       290                 295                 300
   Gln Tyr Asp Asp Ile Val Asp Ser Leu Thr Asn Val Glu Glu Gly Glu
   305                 310                 315                 320
   Glu Lys Asn Met Asn Gly Ser Ala Ser Ala Ala Pro His Lys Glu Glu
   325 330 335
   Arg Gln Gln Phe Leu His Cys Leu Arg Phe Ala Ala Asn Gly Ala Glu
   340 345 350
   Ile Asn Arg Gly Arg Thr Val Trp Arg Arg Lys Leu Ala Arg
   355 360 365
<210> 6
   <211> 363
   <212> PRT
   <213> Sorghum bicolor
<400> 6
   Met Leu Arg Cys His Thr Pro Pro Gln Cys Gly Arg Ala Pro Leu Arg
   1 5 10 15
   His His Gly Arg Arg Glu Ser Pro Pro Ala Ala Ala Pro Gly Val Val
   20 25 30
   Val Arg Cys Ala Arg Gly Ala Pro Gln Val Pro Gly Ile Glu Ala Ala
   35 40 45
   Ser Pro Gly His Ala Ala Ala Thr Ala Ala Lys Ala Glu Gly Gly Asp
   50 55 60
   Ala Arg Pro Ser Leu Ala Glu Arg Leu Arg Leu Gly Ser Leu Leu Glu
   65 70 75 80
   Asp Gly Leu Ser Tyr Lys Glu Ser Phe Ile Val Arg Cys Tyr Glu Val
   85 90 95
   Gly Ile Asn Lys Thr Ala Thr Val Glu Thr Ile Ala Asn Leu Leu Gln
   100 105 110
   Glu Val Gly Cys Asn His Ala Gln Ser Val Gly Phe Ser Thr Asp Gly
   115 120 125
   Phe Ala Thr Thr Thr Thr Met Arg Lys Leu Gly Leu Ile Trp Val Thr
   130 135 140
   Asn Arg Met His Ile Glu Ile Tyr Lys Tyr Pro Ala Trp Gly Asp Val
   145 150 155 160
   Val Glu Ile Glu Thr Trp Cys Gln Glu Asp Gly Arg Ile Gly Thr Arg
   165 170 175
   Arg Asp Trp Ile Leu Lys Asp Leu Ala Asn Gly Glu Val Ile Gly Arg
               180                 185                 190
   Ala Thr Ser Lys Trp Val Met Met Asn Gln Asn Thr Arg Arg Leu Gln
           195                 200                 205
   Arg Val Ser Asp Asp Val Arg Asp Glu Val Phe Ile His Cys Pro Lys
       210                 215                 220
   Thr Pro Arg Leu Ala Phe Pro Glu Glu Asn Asn Gly Ser Leu Lys Lys
   225                 230                 235                 240
   Ile Pro Asn Leu Ser Asp Pro Ala Gln Tyr Ser Arg Leu Gly Leu Val
                   245                 250                 255
   Pro Arg Arg Ala Asp Leu Asp Met Asn Gln His Val Asn Asn Val Thr
               260                 265                 270
   Tyr Ile Gly Trp Val Leu Glu Ser Ile Pro Gln Asp Ile Ile Asp Thr
           275                 280                 285
   His Glu Leu Gln Thr Ile Thr Leu Asp Tyr Arg Arg Glu Cys Gln His
       290                 295                 300
   Asp Asp Ile Val Asp Ser Leu Thr Tyr Ile Glu Glu Gly Glu Glu Lys
   305                 310                 315                 320
   Ser Met Asn Gly Ser Ala Ser Ala Ala Pro His Lys Glu Glu Arg Gln
                   325                 330                 335
   Gln Phe Leu His Cys Leu Arg Phe Ala Ala Asn Gly Asp Glu Ile Asn
               340                 345                 350
   Arg Gly Arg Thr Val Trp Arg Lys Leu Ala Arg
           355                 360
<210> 7
   <211> 362
   <212> PRT
   <213> Arabidopsis thaliana
<400> 7
   Met Leu Lys Leu Ser Cys Asn Val Thr Asp Ser Lys Leu Gln Arg Ser
   1               5                   10                  15
   Leu Leu Phe Phe Ser His Ser Tyr Arg Ser Asp Pro Val Asn Phe Ile
               20                  25                  30
   Arg Arg Arg Ile Val Ser Cys Ser Gln Thr Lys Lys Thr Gly Leu Val
   35 40 45
   Pro Leu Arg Ala Val Val Ser Ala Asp Gln Gly Ser Val Val Gln Gly
   50 55 60
   Leu Ala Thr Leu Ala Asp Gln Leu Arg Leu Gly Ser Leu Thr Glu Asp
   65 70 75 80
   Gly Leu Ser Tyr Lys Glu Lys Phe Val Val Arg Ser Tyr Glu Val Gly
   85 90 95
   Ser Asn Lys Thr Ala Thr Val Glu Thr Ile Ala Asn Leu Leu Gln Glu
   100 105 110
   Val Gly Cys Asn His Ala Gln Ser Val Gly Phe Ser Thr Asp Gly Phe
   115 120 125
   Ala Thr Thr Thr Thr Met Arg Lys Leu His Leu Ile Trp Val Thr Ala
   130 135 140
   Arg Met His Ile Glu Ile Tyr Lys Tyr Pro Ala Trp Gly Asp Val Val
   145 150 155 160
   Glu Ile Glu Thr Trp Cys Gln Ser Glu Gly Arg Ile Gly Thr Arg Arg
   165 170 175
   Asp Trp Ile Leu Lys Asp Ser Val Thr Gly Glu Val Thr Gly Arg Ala
   180 185 190
   Thr Ser Lys Trp Val Met Met Asn Gln Asp Thr Arg Arg Leu Gln Lys
   195 200 205
   Val Ser Asp Asp Val Arg Asp Glu Tyr Leu Val Phe Cys Pro Gln Glu
   210 215 220
   Pro Arg Leu Ala Phe Pro Glu Glu Asn Asn Arg Ser Leu Lys Lys Ile
   225 230 235 240
   Pro Lys Leu Glu Asp Pro Ala Gln Tyr Ser Met Ile Gly Leu Lys Pro
   245 250 255
   Arg Arg Ala Asp Leu Asp Met Asn Gln His Val Asn Asn Val Thr Tyr
   260 265 270
   Ile Gly Trp Val Leu Glu Ser Ile Pro Gln Glu Ile Val Asp Thr His
   275 280 285
   Glu Leu Gln Val Ile Thr Leu Asp Tyr Arg Arg Glu Cys Gln Gln Asp
   290 295 300
   Asp Val Val Asp Ser Leu Thr Thr Thr Thr Ser Glu Ile Gly Gly Thr
   305 310 315 320
   Asn Gly Ser Ala Thr Ser Gly Thr Gln Gly His Asn Asp Ser Gln Phe
   325 330 335
   Leu His Leu Leu Arg Leu Ser Gly Asp Gly Gln Glu Ile Asn Arg Gly
   340 345 350
   Thr Thr Leu Trp Arg Lys Lys Pro Ser Ser
   355 360
<210> 8
   <211> 367
   <212> PRT
   <213> Arabidopsis thaliana
<400> 8
   Met Leu Lys Leu Ser Cys Asn Val Thr Asp His Ile His Asn Leu Phe
   1 5 10 15
   Ser Asn Ser Arg Arg Ile Phe Val Pro Val His Arg Gln Thr Arg Pro
   20 25 30
   Ile Ser Cys Phe Gln Leu Lys Lys Glu Pro Leu Arg Ala Ile Leu Ser
   35 40 45
   Ala Asp His Gly Asn Ser Ser Val Arg Val Ala Asp Thr Val Ser Gly
   50 55 60
   Thr Ser Pro Ala Asp Arg Leu Arg Phe Gly Arg Leu Met Glu Asp Gly
   65 70 75 80
   Phe Ser Tyr Lys Glu Lys Phe Ile Val Arg Ser Tyr Glu Val Gly Ile
   85 90 95
   Asn Lys Thr Ala Thr Ile Glu Thr Ile Ala Asn Leu Leu Gln Glu Val
   100 105 110
   Ala Cys Asn His Val Gln Asn Val Gly Phe Ser Thr Asp Gly Phe Ala
   115 120 125
   Thr Thr Leu Thr Met Arg Lys Leu His Leu Ile Trp Val Thr Ala Arg
   130 135 140
   Met His Ile Glu Ile Tyr Lys Tyr Pro Ala Trp Ser Asp Val Val Glu
   145                 150                 155                 160
   Ile Glu Thr Trp Cys Gln Ser Glu Gly Arg Ile Gly Thr Arg Arg Asp
                   165                 170                 175
   Trp Ile Leu Lys Asp Cys Ala Thr Gly Glu Val Ile Gly Arg Ala Thr
               180                 185                 190
   Ser Lys Trp Val Met Met Asn Gln Asp Thr Arg Arg Leu Gln Arg Val
           195                 200                 205
   Thr Asp Glu Val Arg Asp Glu Tyr Leu Val Phe Cys Pro Pro Glu Pro
       210                 215                 220
   Arg Leu Ala Phe Pro Glu Glu Asn Asn Ser Ser Leu Lys Lys Ile Pro
   225                 230                 235                 240
   Lys Leu Glu Asp Pro Ala Gln Tyr Ser Met Leu Gly Leu Lys Pro Arg
                   245                 250                 255
   Arg Ala Asp Leu Asp Met Asn Gln His Val Asn Asn Val Thr Tyr Ile
               260                 265                 270
   Gly Trp Val Leu Glu Ser Ile Pro Gln Glu Ile Ile Asp Thr His Glu
           275                 280                 285
   Leu Lys Val Ile Thr Leu Asp Tyr Arg Arg Glu Cys Gln Gln Asp Asp
       290                 295                 300
   Ile Val Asp Ser Leu Thr Thr Ser Glu Thr Pro Asn Glu Val Val Ser
   305                 310                 315                 320
   Lys Leu Thr Gly Thr Asn Gly Ser Thr Thr Ser Ser Lys Arg Glu His
                   325                 330                 335
   Asn Glu Ser His Phe Leu His Ile Leu Arg Leu Ser Glu Asn Gly Gln
               340                 345                 350
   Glu Ile Asn Arg Gly Arg Thr Gln Trp Arg Lys Lys Ser Ser Arg
           355                 360                 365
<210> 9
   <211> 1092
   <212> DNA
   <213> Artificial
<220>
   <223> séquence synthétique codant ZmFatA
<400> 9
   atgctgcgtt gccatgcacc accccagtgc ggtcgggctc ctctgcgcca tcacgggagg      60
   tgggagagta gcccagcccc tggtgttgtg gtgcgctgca ccagaggcgc accccaagtg     120
   tccggcatag aggcggccag tccggaccat gccgctgcga ctgccgtcgc ggcaaaggct     180
   gagggcggag atgcgaggcc ctcccttgct gagaggctca gattgggctc gcttctggag     240
   gacgggctct cttacaagga gtcgttcata gtccgctgct acgaggtggg gatcaacaaa     300
   accgcaacgg tggaaaccat cgccaatctg ctgcaagagg ttggatgcaa tcacgcacag     360
   tccgtgggat tttctacgga  cgggttcgct acgacgacga ccatgagaaa gcttggtctt     420
   atttgggtga ctaaccgcat gcacattgag atttataagt atccggcgtg gggagatgtc     480
   gtcgagatcg agacatggtg tcaagaagat ggcaagatcg ggaccaggcg cgattggatc     540
   ctcaaggacc tctgtacagg cgaagtcacc ggcagggcta catctaagtg ggttatgatg     600
   aatcaaaaca ctagaagatt gcagcgggtg agtgatgacg tccgcgatga agtgtttatc     660
   cattgtccga aaacaccacg  gctggcgttc ccggaagaga acaatggttc gctcaaaaag     720
   atccctaact tgtcagatcc tgcccaatac tcaagactgg gcttggtgcc caggagggcc     780
   gacctcgaca tgaaccagca tgtcaacaat gttacttaca ttggctgggt tttggagagc     840
   attccgcagg acattataga tacccacgaa ctgcaaacga tcactcttga ttaccgtcgg     900
   gaatgtcagc acgatgacat tgtcgactca ctgacctatg tggaggaagg ggaggaacgt     960
   agcatgaacg gctcagcttc cagcgtccca cacactgagc agcgcaggca attcctccac    1020
   tgtctgcgct tcgccgccaa cggtgatgaa atcaaccggg gtcggacagt ttggcgcaag    1080
   ctcgcgaggt ga                                                        1092

## Description

La présente invention concerne un procédé d'obtention de plantes tolérantes à un déficit hydrique.

Le « déficit hydrique » correspond à une situation où la quantité d'eau transpirée par une plante est supérieure à la quantité d'eau absorbée par ladite plante.

Le déficit hydrique est l'un des stress abiotiques les plus importants pour les plantes. Il peut affecter leur croissance et leur reproduction des plantes, conduisant ainsi à une perte de rendement.

Par conséquent, il est important d'identifier des gènes qui possèdent la capacité d'améliorer la tolérance des plantes au déficit hydrique.

Les protéines ENR (« *enoyl-acyl carrier protein (ACP) reductase* ») et TE (acyl-ACP thioestérase, E.C. 3.1.2.14) sont impliquées dans la biosynthèse des acides gras chez les plantes. Les protéines ENR convertissent le 2,3-*trans*-enoyl-ACP en acyl-ACP saturé et les protéines TE hydrolysent la moitié acyl de l'ACP libérant un ACP-SH et un acide gras qui va subir d'autres transformations *via* la voie de biosynthèse des acides gras à longues chaînes catalysée par l'acyl-CoA synthétase. Il a été montré que les protéines ENR et TE interagissent physiquement ensemble (HELLYER et al., Plant Mol. Biol., 20:763-780, 1992). Elles constitueraient un métabolon facilitant le transfert de substrats et de produits, et la régulation simultanée (« *channeling* ») des enzymes impliquées dans la voie métabolique des acides gras (BROWN et al., J. Exp. Bot., 57:1563-1571, 2006).

Il existe deux classes majeurs de protéines TE : la classe de type FatA, qui a comme substrat préférentiel l'acide oléique-ACP (18:1-ACP), et la classe de type FatB qui a comme substrat préférentiel l'acide palmitique-ACP (16:0-ACP) (BEISSON et al., Plant Physiol., 132:681-697, 2003).

Chez le maïs, une protéine TE de type FatA (dénommée ZmFatA), dont le gène codant pour cette protéine est localisé sur le chromosome 2, possède la séquence peptidique représentée par la séquence SEQ ID NO : 1.

MAYER et SHANKLIN (BMC Plant Biology, 7:1, 2007) ont identifié 4 résidus d'acides aminés dans la séquence peptidique des protéines TE d'*Arabidospsis thaliana* (acides aminés en position 74, 86, 141 et 174) qui influencent, chez les plantes, la spécificité de substrat entre les protéines TE de type FatA et celles de type FatB. Ces 4 résidus sont respectivement situés aux positions 108, 120, 175 et 208 dans la séquence d'acides aminés de ZmFatA (SEQ ID NO : 1).

Au cours de leurs travaux, les inventeurs ont mis en évidence que des plantes de maïs (*Zea mays*) transgéniques surexprimant la protéine TE de type FatA (ZmFatA) présentaient une tolérance augmentée à un déficit hydrique par rapport aux plantes de maïs sauvages (non transgéniques).

Les inventeurs ont également mis en évidence les orthologues de la protéine ZmFatA chez le riz (*Oryza sativa*), le sorgho (*Sorghum bicolor*), le blé (*Triticum aestivum*), *Brachypodium distachyon* et *Arabidopsis thaliana.*

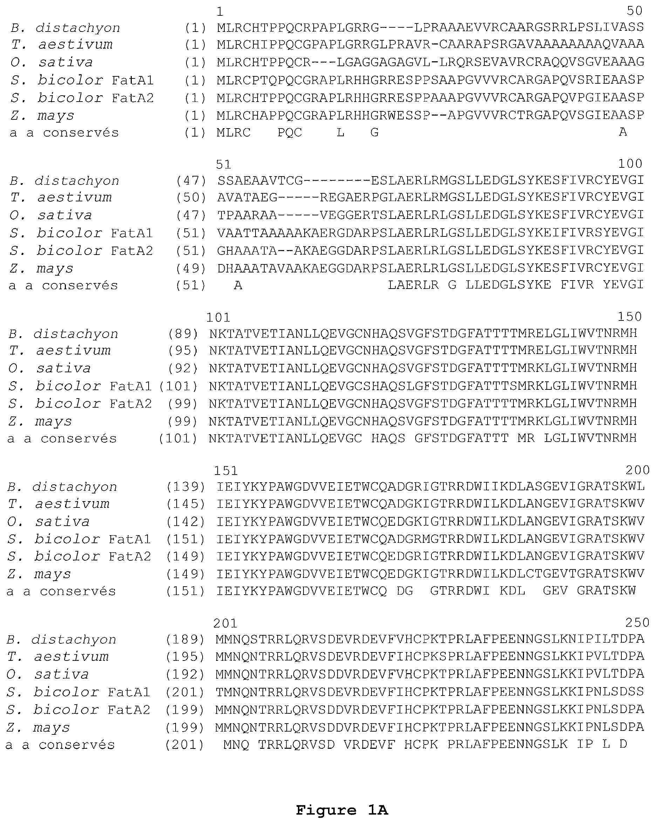

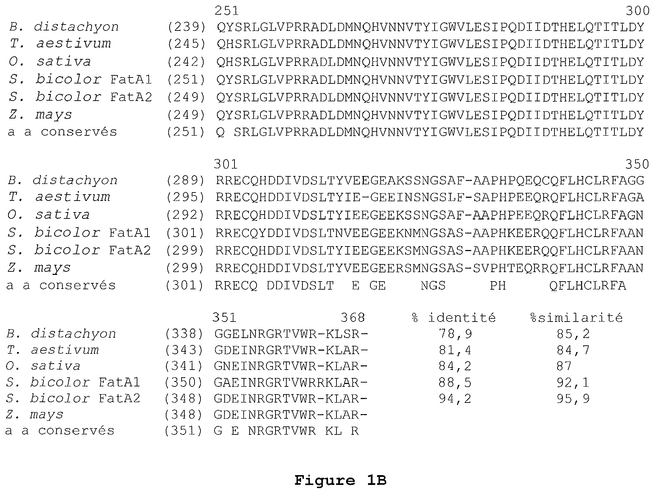

La séquence peptidique des orthologues chez les plantes monocotylédones (e.g., sorgho, blé et *Brachypodium*) possède au moins 78% d'identité ou au moins 84% de similarité avec la séquence peptidique ZmFatA et comprend les acides aminés conservés situés aux positions 1-4, 8-10, 15, 19, 46, 51, 69-74, 76-87, 89-92, 94-116, 118-121, 123-133, 135-136, 138-168, 170-171, 174-180, 182-184, 187-189, 191-197, 200-202, 204-212, 214-219, 221-224, 226-239, 241-242, 244, 246, 249, 251-303, 305-312, 315, 317-318, 323-325, 330-331, 337-345, 348, 350, 352-359, 360-361 et 363 de ZmFatA (SEQ ID NO : 1) lorsqu'elle est alignée avec la protéine ZmFatA (voir les Figures 1A et 1B).

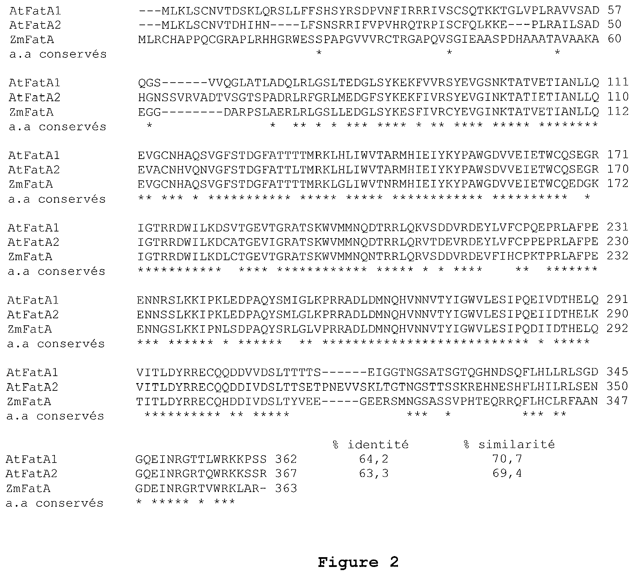

La séquence peptidique des orthologues chez A. *thaliana* (plante dicotylédone) (BEISSON et *al.,* 2003, cité ci-dessus) possède au moins 63% d'identité ou au moins 69% de similarité avec la séquence peptidique ZmFatA et comprend les acides aminés conservés situés aux positions 24, 55, 61, 70, 73-74, 76, 78, 80-82, 84-87, 89, 91-92, 94-97, 99-103, 105-114, 116-118, 120, 122-132, 134-138, 140-144, 146-157, 159-168, 171, 173-183, 186-189, 191-202, 204-208, 210, 212, 214-217, 222-223, 226-235, 237-242, 244, 246-251, 254-255, 257-283, 285, 287-291, 294-303, 305-306, 308-312, 323-325, 328, 338-340, 342-343, 348, 350-354, 356 et 358-360 de ZmFatA (SEQ ID NO : 1) lorsqu'elle est alignée avec la protéine ZmFatA (voir Figure 2).

Sauf précision contraire, l'alignement entre deux séquences peptidiques et le calcul des pourcentages d'identité et de similarité sont effectués sur toute la longueur des séquences peptidiques à l'aide du programme d'ordinateur *« needle »* (NEEDLEMAN et WUNSCH, J. Mol. Biol., 48, 443-453, 1970) en utilisant les paramètres par défaut : *« Matrix* » : EBLOSUM62, « *Gap penalty* » : 10,0 et « *Extend penalty* » : 0,5.

La présente invention propose en conséquence d'utiliser la protéine ZmFatA ou un orthologue de celle-ci pour augmenter la résistance des plantes au déficit hydrique.

La présente invention a pour objet un procédé pour augmenter la tolérance d'une plante au déficit hydrique, caractérisé en ce que l'on surexprime dans ladite plante une protéine TE de type FatA possédant au moins 60% d'identité, et par ordre de préférence croissant au moins 65%, 70%, 75%, 80%, 85%, 90%, 95%, 97%, 98% et 99% d'identité, ou au moins 70% de similarité, et par ordre de préférence croissant au moins 75%, 80%, 85%, 90%, 95%, 97%, 98% et 99% de similarité avec la séquence SEQ ID NO: 1.

Selon un mode de réalisation avantageux de la présente invention, ladite protéine TE de type FatA comprend les acides aminés conservés situés aux positions 70, 73-74, 76, 78, 80-82, 84-87, 89, 91-92, 94-97, 99-103, 105-114, 116, 118, 120, 123-132, 135-136, 138, 140-144, 146-157, 159-168, 171, 174-180, 182-183, 187-189, 191-197, 200-202, 204-208, 210, 212, 214-217, 222-223, 226-235, 237-239, 241-242, 244, 246, 249, 251, 254-255, 257-283, 285, 287-291, 294-303, 305-306, 308-312, 323-325, 338-340, 342-343, 348, 350, 352-354, 356, 358-359 et 360 de ladite séquence SEQ ID NO : 1 lorsqu'elle est alignée avec ladite séquence SEQ ID NO : 1.

Selon un autre mode de réalisation avantageux de la présente invention, ladite protéine TE de type FatA est issue d'une plante monocotylédone, et possède au moins 78% d'identité, et par ordre de préférence croissant au moins 80%, 85%, 90%, 95%, 97%, 98% et 99% d'identité, ou au moins 84% de similarité, et par ordre de préférence croissant au moins 85%, 90%, 95%, 97%, 98% et 99% de similarité avec la séquence SEQ ID NO: 1 et comprend les acides aminés conservés situés aux positions 1-4, 8-10, 15, 19, 46, 51, 69-74, 76-87, 89-92, 94-116, 118-121, 123-133, 135-136, 138-168, 170-171, 174-180, 182-184, 187-189, 191-197, 200-202, 204-212, 214-219, 221-224, 226-239, 241-242, 244, 246, 249, 251-303, 305-312, 315, 317-318, 323-325, 330-331, 337-345, 348, 350, 352-359, 360-361 et 363 de ladite séquence SEQ ID NO : 1 lorsqu'elle est alignée avec ladite séquence SEQ ID NO : 1.

Selon un autre mode de réalisation avantageux de la présente invention, ladite protéine TE de type FatA est issue d'une plante dicotylédone, et comprend les acides aminés conservés situés aux positions 24, 55, 61, 70, 73-74, 76, 78, 80-82, 84-87, 89, 91-92, 94-97, 99-103, 105-114, 116-118, 120, 122-132, 134-138, 140-144, 146-157, 159-168, 171, 173-183, 186-189, 191-202, 204-208, 210, 212, 214-217, 222-223, 226-235, 237-242, 244, 246-251, 254-255, 257-283, 285, 287-291, 294-303, 305-306, 308-312, 323-325, 328, 338-340, 342-343, 348, 350-354, 356 et 358-360 de ladite séquence SEQ ID NO : 1 lorsqu'elle est alignée avec ladite séquence SEQ ID NO : 1.

On entend par une protéine TE de type FatA issue d'une plante monocotylédone ou dicotylédone, une protéine TE de type FatA exprimée par une plante monocotylédone ou dicotylédone ou une protéine TE de type FatA synthétique obtenue par mutation d'une protéine TE de type FatA exprimée par une plante monocotylédone ou dicotylédone.

Ladite protéine TE de type FatA est fonctionnelle. La détermination d'une protéine TE de type FatA fonctionnelle peur être effectuée selon la méthode décrite par MAYER et SHANKLIN (BMC Plant Biology, 7:1, 2007). Brièvement, un plasmide contenant un gène codant pour une protéine TE de type FatA telle que définie ci-dessus est introduit dans la souche *d'E. coli* K27 (CGSC 5478). Les souches d'E. *coli* transformées sont ensuite cultivées dans un milieu approprié. La teneur et la quantité des esters méthyliques d'acides gras excrétés dans le milieu de culture par lesdites souches sont alors déterminées.

Selon un autre mode de réalisation préféré de la présente invention, ladite protéine TE de type FatA est choisie dans le groupe constitué par les séquences d'acides aminés suivantes :
- SEQ ID NO : 1 (ZmFatA),
- SEQ ID NO : 2 (protéine TE de type FatA de *B. distachyon*),
- SEQ ID NO : 3 (protéine TE de type FatA de *T. aestivum*),
- SEQ ID NO : 4 (protéine TE de type FatA d'O. *sativa*),
- SEQ ID NO : 5 et 6 (protéines TE de type FatA de *S. bicolor*), et
- SEQ ID NO : 7 et 8 (protéines TE de type FatA *d'A. thaliana*).
- SEQ ID NO : 8 (protéine TE de type FatA d'A. *thaliana*), de préférence encore, SEQ ID NO : 1.

La présente invention s'applique à toutes les plantes monocotylédones ou dicotylédones, et notamment aux plantes sensibles au déficit hydrique. De manière non limitative, elle peut s'appliquer aux plantes potagères, aux plantes ornementales, aux arbres fruitiers, aux plantes de grandes cultures telles que le blé, le maïs ou le riz, ou aux plantes de cultures industrielles comme le cotonnier, le colza ou le tournesol, de préférence le maïs.

La surexpression (augmentation de l'expression) dans une plante d'une protéine TE de type FatA telle que définie ci-dessus, peut être effectuée par la modification du génome de ladite plante. Cette modification du génome peut notamment s'effectuer par transformation génétique de ladite plante par une ou plusieurs copies d'un polynucléotide codant pour ladite protéine, associé à des séquences de régulation en *cis* de son expression. La surexpression de ladite protéine TE de type FatA peut également être obtenue par modification des séquences de régulation en *cis* de l'expression de ladite protéine TE FatA, par exemple en remplaçant son promoteur endogène par un promoteur plus fort, permettant un niveau de transcription plus élevé, ou bien en adjoignant au promoteur endogène des séquences activatrices de la transcription, de type « amplificateur », ou de la traduction.

Pour la mise en oeuvre du procédé selon la présente invention, on utilise une cassette d'expression, comprenant un polynucléotide codant pour une protéine TE de type FatA telle que définie ci-dessus, placé sous le contrôle transcriptionnel d'un promoteur approprié.

Ledit promoteur peut être un promoteur hétérologue. Dans ce cas, on peut utiliser par exemple :
- des promoteurs constitutifs, tels que le promoteur de la gluténine de haut poids moléculaire spécifique de l'endosperme (VERDAGUER et al., Plant Mol Biol., 31:1129-1139, 1996), le promoteur de l'ARN 35S (ODELL et al., Nature, 313:810-812, 1985) ou 19S (KAY et al., Science, 236:1299-1302, 1987) du CaMV, le promoteur de l'actine 1 du riz (McELROY et al., Plant Cell, 2:163-171, 1990), le promoteur de l'ubiquitine 3 du riz ou du maïs (SIVAMANI et QU, Plant Mol Biol., 60:225-239, 2006),
- des promoteurs spécifiques du phloème, tels que le promoteur du Wheat Dwarf Virus (DINANT et al., Physiologia plantarum 121 : 108-116, 2004 ; Demande PCT WO 03/060135) ou le promoteur de *AtPP2-A1* (DINANT et al., Plant Physiol., 131 : 114-128, 2003),
- des promoteurs spécifiques des feuilles, tels que le promoteur de la petite sous-unité de la Rubisco, le promoteur de la phosphoénolpyruvate carboxylase,
- des promoteurs spécifiques des racines, tels que le promoteur RCc3 du riz (Demande Internationale WO 2009/016104), le promoteur antiquitine du riz (Demande Internationale WO 2007/076115), ou
- des promoteurs localement inductibles par le stress (sécheresse, salinité), tels que le promoteur rd29 d'*Arabidopsis* (YAMAGUCHI-SHINOZAKI et SHINOZAKI, Mol. Gen. Genet., 236: 331-340, 1993),
de préférence le promoteur de la gluténine de haut poids moléculaire spécifique de l'endosperme.

On peut également utiliser le promoteur d'une autre protéine TE (par exemple une protéine TE de type FatB).

Pour la mise en oeuvre du procédé selon la présente invention, on peut aussi utiliser des vecteurs recombinants, résultant de l'insertion d'une cassette d'expression telle que décrite ci-dessus dans un vecteur hôte.

Les cassettes d'expression et vecteurs recombinants tels que décrits ci-dessus peuvent, bien entendu, comprendre en outre d'autres séquences, usuellement employées dans ce type de constructions. Le choix de ces autres séquences sera effectué, de manière classique, par l'homme du métier en fonction notamment de critères tels que les cellules-hôtes choisies, les protocoles de transformation envisagés, etc.

On citera, à titre d'exemples non limitatifs, les terminateurs de transcription, les séquences de tête (séquences « *leader »)* et les sites de polyadénylation. Ces séquences peuvent être celles qui sont naturellement associées au gène codant la protéine TE de type FatA telle que définie ci-dessus, ou bien peuvent être des séquences hétérologues. Ces séquences n'interviennent pas sur les propriétés spécifiques du promoteur ou du gène auxquelles elles sont associées, mais peuvent améliorer globalement qualitativement ou quantitativement, la transcription, et le cas échéant, la traduction. A titre d'exemples de séquences de ce type fréquemment utilisées chez les plantes, on citera parmi les plus répandues, le terminateur de l'ARN 35S du CaMV et le terminateur du gène de la nopaline synthase. On peut également, dans le but d'augmenter le niveau d'expression, utiliser des séquences amplificatrices (séquences « *enhancer* » de transcription et de traduction).

Parmi les autres séquences couramment employées dans la construction de cassettes d'expression et vecteurs recombinants, on citera également les séquences permettant le suivi de la transformation, et l'identification et/ou la sélection des cellules ou organismes transformés. Il s'agit notamment de gènes rapporteurs, conférant aux cellules ou organismes transformés un phénotype aisément reconnaissable, ou bien de gènes marqueurs de sélection : seules les cellules ou organismes exprimant un gène marqueur de sélection déterminé, sont viables dans des conditions données (conditions sélectives). Des gènes rapporteurs fréquemment employés sont par exemple celui de la beta-glucuronidase (GUS), celui de la luciférase, ou celui de la « *green fluorescent protein »* (GFP). Des gènes marqueurs de sélection sont généralement des gènes de résistance à un antibiotique, ou également, dans le cas des plantes ou des cellules végétales, à un herbicide. Il existe une très grande variété de gènes marqueurs de sélection parmi lesquels l'homme du métier peut effectuer son choix en fonction des critères qu'il aura lui-même déterminés.

Pour la mise en oeuvre du procédé selon la présente invention, on peut également utiliser des cellules-hôtes transformées par un polynucléotide codant pour une protéine TE de type FatA telle que définie ci-dessus, ce qui inclut en particulier les cellules hôtes transformées par une cassette d'expression ou un vecteur recombinant tels que décrits ci-dessus.

On entend par cellule ou organisme transformé par un polynucléotide, toute cellule ou organisme dont le contenu génétique a été modifié par transfert dudit polynucléotide dans ladite cellule ou ledit organisme, quelle que soit la méthode de transfert qui a été utilisée, et que l'information génétique apportée par ledit polynucléotide soit intégrée dans l'ADN chromosomique ou demeure extra-chromosomique.

Les cellules hôtes peuvent être des cellules procaryotes ou eucaryotes. Dans le cas de cellules procaryotes, il peut notamment s'agir d'Agrobactéries telles qu' *Agrobacterium tumefaciens ou Agrobacterium rhizobium.* Dans le cas de cellules eucaryotes, il peut s'agir notamment de cellules végétales, issues de plantes monocotylédones ou dicotylédones.

Les plantes transgéniques peuvent être obtenues par transformation génétique par au moins un polynucléotide, une cassette d'expression ou un vecteur recombinant tels que définis ci-dessus. Ces plantes transgéniques comprennent dans leur génome au moins une copie d'un transgène contenant un polynucléotide tel que défini ci-dessus.

On définit ici comme plante transgénique une plante transformée chez laquelle l'information génétique exogène apportée par un polynucléotide transformant est intégrée de manière stable dans l'ADN chromosomique, sous forme de transgène, et peut ainsi être transmise aux descendants de ladite plante. Cette définition englobe donc également les descendants des plantes résultant de la transgénèse initiale, dès lors qu'ils contiennent dans leur génome une copie du transgène.

Le matériel végétal (protoplastes, cals, boutures, graines, etc.) obtenu à partir des cellules transformées ou des plantes transgéniques fait également partie de l'objet de la présente invention. L'invention englobe également les produits obtenus à partir de ces plantes transgéniques, notamment le fourrage, le bois, les feuilles, les tiges, les racines, les fleurs et les fruits.

Différentes méthodes d'obtention de plantes transgéniques sont bien connues en elles-mêmes de l'homme du métier. Généralement, ces méthodes impliquent la transformation de cellules végétales, la régénération de plantes à partir des cellules transformées, et la sélection des plantes ayant intégré le transgène.

De nombreuses techniques de transformation de cellules végétales germinales ou somatiques (isolées, sous forme de cultures de tissus ou d'organe, ou sur la plante entière), et de régénération des plantes sont disponibles. Le choix de la méthode la plus appropriée dépend généralement de la plante concernée.

A titre d'exemples non limitatifs de méthodes utilisables dans le cas des plantes mentionnées ci-dessus, il est possible de citer les protocoles décrits par GUIS et al. (Scientia Horticulturae 84 : 91-99, 2000) pour le melon, par HAMZA et CHUPEAU (J. Exp. Bot. 44 : 1837-1845, 1993) pour la tomate, par SHOEMAKER et al. (Plant Cell Rep. 3 : 178-181, 1986), ou TROLINDER et GOODIN (Plant Cell Rep. 6 : 231-234, 1987) pour le cotonnier, par VAN DER MARK et al. (J. Genet Breeding 44 : 263-268, 1990) ou par MARCHANT et al. (Ann. Bot. 81 : 109-114, 1998) pour les rosiers. Dans le cas des plantes monocotylédones, on peut citer par exemple les protocoles décrits par HIEI et al. (The Plant Journal, 6, 271-282, 1994) ou ISHIDA et al. (Nature biotechnology, 14, 745-750, 1996) pour le maïs, ou par RASCO-GAUNT et al. (J. Exp. Bot. 52 : 865-874, 2001) pour le blé.

La présente invention sera mieux comprise à l'aide du complément de description qui va suivre, qui se réfère à des exemples non-limitatifs illustrant l'obtention de plantes transgéniques surexprimant la protéine TE de type FatA telle que définie ci-dessus et la mise en évidence de son rôle dans l'augmentation de la résistance au déficit hydrique, ainsi que des figures annexées :
**Figure 1A** **et** **1B** : alignement et pourcentage d'identité et de similarité des séquences peptidiques des protéines TE de type FatA de *B. distachyon* (SEQ ID NO : 2), *T. aestivum* (SEQ ID NO : 3), d'*O*. *sativa* (SEQ ID NO : 4), de *S. bicolor* (SEQ ID NO : 5, FatA1 et SEQ ID NO : 6, FatA2) avec la protéine TE de type FatA de *Z*. *mays* (ZmFatA, SEQ ID NO : 1), en utilisant le programme d'ordinateur « *needle » (« Matrix* » : EBLOSUM62, *« Gap penalty* » : 10,0 et *« Extend penalty* » : 0,5). Les acides aminés conservés (a. a. conservés) entre toutes les séquences peptidiques sont aussi représentés.
**Figure 2** : alignement et pourcentage d'identité et de similarité des séquences peptidiques des protéines TE de type FatA d'A. *thaliana* (SEQ ID NO : 7 [AtFatA1] et SEQ ID NO : 8 [AtFatA2]) avec la protéine TE de type FatA de *Z. mays* (ZmFatA, SEQ ID NO : 1), en utilisant le programme d'ordinateur *« needle » (« Matrix* » : EBLOSUM62, *« Gap penalty* » : 10,0 et *« Extend penalty* » : 0,5). Les acides aminés conservés (a. a. conservés) entre toutes les séquences peptidiques sont représentés par un astérisque (*).

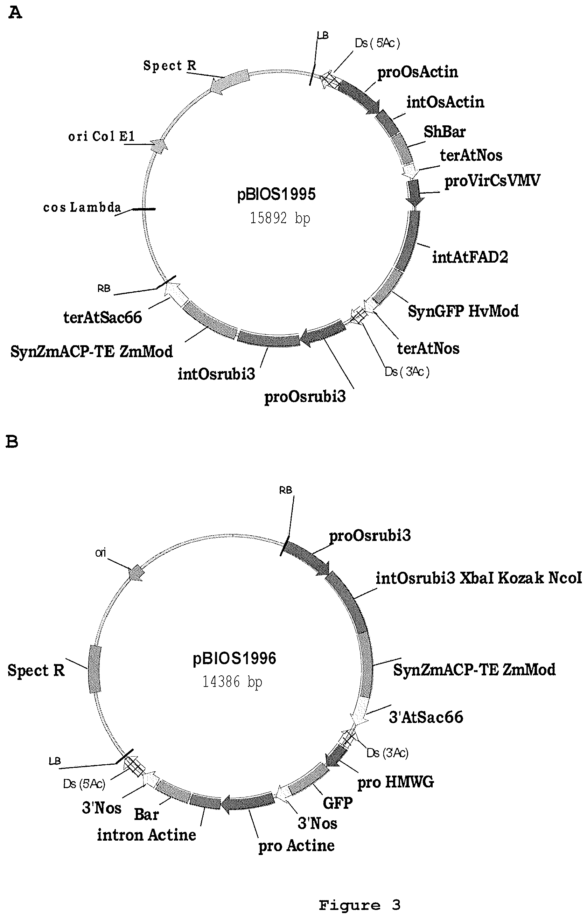

**Figure 3** : carte des vecteurs binaires pBIOS1996 (**A**) et pBIOS1995 (**B**).

### EXEMPLE 1 : OBTENTION DE MAÏS TRANSGENIQUES SUREXPRIMANT LA PROTEINE ZmFatA

### 1) Clonage et transformation génétique du maïs

Deux vecteurs de transformation différents (pBIOS 1562 et pBIOS 1958) ont été utilisés pour la transformation génétique du maïs. Ces vecteurs contiennent le gène bar de *Streptomyces hygroscopicus* conférant la résistance à l'herbicide bialaphos (WHITE et al., Nucleic Acids Res., 18:1062, 1990), utile pour la sélection des transformants de maïs, et un gène codant une GFP (*Green Fluorescente Protein*) en tant que marqueur visuel pour suivre la présence du transgène dans les plantes et les graines. La différence entre ces deux vecteurs réside dans la stratégie de clonage utilisée pour introduire la cassette d'expression contenant le gène d'intérêt (clonage via le système Gateway® ou clonage par restriction) et le promoteur pour l'expression de la GFP (le promoteur du virus de la mosaïque des nervures du manioc (CsVMV) suivi par l'intron FAD2 d'*Arabidopsis* ou le promoteur de la gluténine de haut poids moléculaire spécifique de l'endosperme).

Selon une première stratégie de clonage, le gène synthétique codant ZmFatA (SEQ ID NO : 9 ; séquence synthétique optimisée pour l'expression dans le maïs) contenant les sites de restriction *att*L1 et *att*L2 a été introduit par une réaction de recombinaison LR dans le vecteur binaire de destination GATEWAY pBIOS 1562, générant ainsi le vecteur pBIOS1995 (voir Figure 3A). Le vecteur pBIOS 1562 est dérivé du vecteur pSB12 (KOMARI et al., Plant J., 10:165-174, 1996) contenant le gène bar sous le contrôle du promoteur pActin, le gène codant une GFP sous le contrôle du promoteur du CsVMV suivi par l'intron FAD2, le promoteur et le 1^{er} intron de l'ubiquitine 3 du riz (SIVAMANI et QU, Plant Mol Biol., 60:225-239, 2006) suivie d'une cassette GATEWAY et d'une séquence de polyadénylation provenant du gène Sac66 d'*Arabidopsis* (JENKINS et al., Plant Cell Environ., 22:159-167, 1999).

Selon une seconde stratégie de clonage, le gène synthétique codant ZmFatA (SEQ ID NO : 9) a été introduit par clonage par restriction (présence de sites de restriction *SapI* entre la région codante et les sites attL) dans le vecteur binaire de destination pBIOS 1958 digéré par *SapI,* générant ainsi le vecteur pBIOS1996 (voir Figure 3B). pBIOS 1958 est également dérivé du vecteur pSB12, mais possède le gène codant une GFP sous le contrôle du promoteur de la gluténine de haut poids moléculaire spécifique de l'endosperme (promoteur HMWG).

les vecteurs pBIOS1995 ou pBIOS1996 ont ensuite été transférés dans la souche *d'Agrobacterium tumefaciens* LBA4404 (pSB1) selon la méthode décrite par KOMARI *et al*., 1996 (cité ci-dessus).

Le cultivar de maïs A188 a ensuite été transformé par cette souche d'agrobactérie contenant le vecteur pBIOS1995 ou le vecteur pBIOS1996, selon la méthode décrite par ISHIDA *et al.,* 1996 (cité ci-dessus). Les transformants primaires (T0) ont été sélectionnés selon des méthodes de routine en fonction des quatre critères suivants :
(i) *nombre de copies insérées* : Cette détermination a été réalisée par PCR quantitative. Tous les évènements de transformation possédant plus de 2 copies du transgène ont été éliminés.
(ii) *intégrité de l'ADN-T inséré* : Elle a été vérifiée par une réaction de PCR au cours des premières étapes de développement de la plante transformée.
(iii) *absence de terminaison prématurée de la transcription du transgène* : Chacun des gènes ciblés étant sous le contrôle d'un promoteur constitutif, il est possible de mesurer leur expression à partir de tissus foliaires. Les ARN de feuilles de plantes T0 ont donc été extraits et l'intégrité des transcrits vérifiée par RT-PCR à l'aide d'une amorce sens située sur l'intron ubiquitine3 de riz et d'une amorce antisens située sur le terminateur AtSac66.
(iv) *nombre de grains T1 récoltés.*

Après sélection des transformants, il a été obtenu 41 lignées trangéniques dont 18 ont un transgène unique et intègre.

### 2) Evaluation de la tolérance des plantes transgéniques au déficit hydrique

Des plantes de première génération (croisement du transformant primaire avec la lignée récurrente A188) sont évaluées sur une plate-forme de phénotypage. Ces plantes transgéniques sont donc hémizygotes pour le transgène (caractère dominant de la transformation génétique). Les témoins (« RRS » et « RCP) utilisés dans l'expérimentation correspondent aux ségrégants sauvages issus de ce même croisement.

### 2.1 Compartiment de culture

Les plantes étudiées sont cultivées en phytotron. Ce dernier, d'une surface de 30 m2 dispose de deux chambres de culture indépendantes. Dans ces chambres l'éclairage, la température et l'hygrométrie sont régulés (voir paragraphe 2.2. ci-dessous)

Les semis sont réalisés dans des terrines de dimensions 44 x 28,5 x 7 cm (H x 1 x L). Cinq génotypes sont semés par terrine à raison de dix graines par génotype. Cinq plantes seulement par génotype sont utilisées pour la cinétique de dessèchement.

### 2.2 Conditions de culture

Au sein du compartiment de culture la température, l'humidité et l'éclairage sont régulés.

Les conditions appliquées sont les suivantes :

### Photopériode :

- Jour durant 16h (6h à 22h) avec complément photosynthétique (lampe à sodium 400W) lorsque le rayonnement extérieur est inférieur à 100W/m².
- Nuit durant 8h (22h à 6h).
*Thermopériode :* 24°C / 20°C.

Le respect de ces conditions est assuré par un chauffage lorsque la température est inférieure à 20°C la nuit ou 24°C le jour, lorsque la température dépasse 25°C.
*Humidité :* 75% d'humidité relative régulée par brumisation nocturne.

Ces différentes conditions assurent une croissance optimale du maïs.

### 2.3 Mesure de la cinétique de dessèchement

### Pertinence du caractère mesuré :

Le comportement des plantes vis-à-vis de la transpiration est étudié grâce au suivi en continu de la baisse de la teneur en eau relative (RWC : Relative Water Content) de plantules à un stade jeune (4 feuilles visibles). L'objectif est d'étudier la réponse en terme de contrôle stomatique des plantes lors d'un arrêt brutal de l'alimentation en eau.

Un contrôle stomatique très rapide à l'apparition d'un déficit hydrique permet d'économiser l'eau disponible mais limite la capacité d'assimilation du CO₂ donc le potentiel de production de la plante. En revanche, une fermeture assez tardive des stomates permet le maintien de l'activité photosynthétique de la plante assurant le maintien du potentiel de production au risque de se dessécher plus rapidement (Khalfaoui, 1991).

### Méthode :

Les mesures sont réalisées sur des plantules T1 entières au stade 3-4 feuilles visibles. Les plantes utilisées au cours de cette mesure sont des plantes issues d'un semis en excès par rapport aux besoins de la plate-forme (3 graines semées par pot). Les effectifs pour la mesure de dessèchement sont de 5 plantes par évènement de transformation et témoins sauvages.

Les plantes sont coupées au niveau du collet, immergées pendant 24 heures à 4°C à l'obscurité (pour saturer les cellules en eau) puis placées dans une enceinte climatique lumineuse régulée à 30°C.

Un suivi du poids des plantules est alors réalisé selon le calendrier détaillé dans le Tableau I ci-dessous :

Tableau I : Calendrier des pesées des plantules conditionnées à 30°C en pleine lumière. Le poids à H0 correspond au poids à pleine turgescence. En fin de jour 3, les plantules sont placées dans une étuve à 80°C pendant 24h00 afin d'obtenir par une dernière pesée la valeur de poids sec.

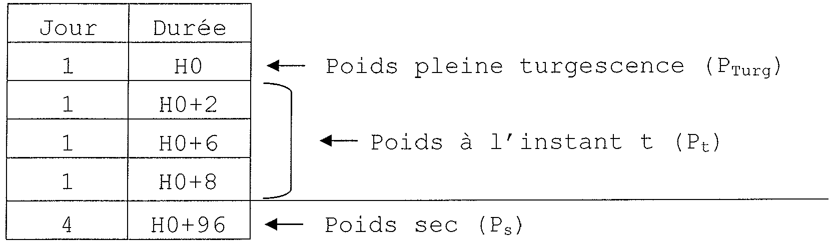

A l'instant t, la teneur en eau relative des plantes est ensuite calculée selon la formule mathématique suivante : (Pₜ-Pₛ) / (P_{Turg}- Pₛ) x 100.
## Revendications

1. Procédé pour augmenter la tolérance d'une plante au déficit hydrique, **caractérisé en ce que** l'on surexprime dans ladite plante une protéine TE de type FatA possédant au moins 60% d'identité ou au moins 70% de similarité avec la séquence SEQ ID NO: 1.

2. Procédé selon la revendication 1, **caractérisé en ce que** ladite protéine TE de type FatA comprend les acides aminés conservés situés aux positions 70, 73-74, 76, 78, 80-82, 84-87, 89, 91-92, 94-97, 99-103, 105-114, 116, 118, 120, 123-132, 135-136, 138, 140-144, 249, 146-157, 159-168, 171, 174-180, 182-183, 187-189, 191-197, 200-202, 204-208, 210, 212, 214-217, 222-223, 226-235, 237-239, 241-242, 244, 246, 249, 251, 254-255, 257-283, 285, 287-291, 294-303, 305-306, 308-312, 323-325, 338-340, 342-343, 348, 350, 352-354, 356, 358-359 et 360 de ladite séquence SEQ ID NO : 1 lorsqu'elle est alignée avec ladite séquence SEQ ID NO : 1.

3. Procédé selon la revendication 2, **caractérisé en ce que** ladite protéine TE de type FatA est issue d'une plante monocotylédone, et possède au moins 79% d'identité, ou au moins 79% de similarité avec la séquence SEQ ID NO: 1, et comprend les acides aminés conservés situés aux positions 1-4, 8-10, 15, 19, 46, 51, 69-74, 76-87, 89-92, 94-116, 118-121, 123-133, 135-136, 138-168, 170-171, 174-180, 182-184, 187-189, 191-197, 200-202, 204-212, 214-219, 221-224, 226-239, 241-242, 244, 246, 249, 251-303, 305-312, 315, 317-318, 323-325, 330-331, 337-345, 348, 350, 352-359, 360-361 et 363 de ladite séquence SEQ ID NO : 1 lorsqu'elle est alignée avec ladite séquence SEQ ID NO : 1.

4. Procédé selon la revendication 2, **caractérisé en ce que** ladite protéine TE de type FatA est issue d'une plante dicotylédone, et comprend les acides aminés conservés situés aux positions 24, 55, 61, 70, 73-74, 76, 78, 80-82, 84-87, 89, 91-92, 94-97, 99-103, 105-114, 116-118, 120, 122-132, 134-138, 140-144, 146-157, 159-168, 171, 173-183, 186-189, 191-202, 204-208, 210, 212, 214-217, 222-223, 226-235, 237-242, 244, 246-251, 254-255, 257-283, 285, 287-291, 294-303, 305-306, 308-312, 323-325, 328, 338-340, 342-343, 348, 350-354, 356 et 358-360 de ladite séquence SEQ ID NO : 1 lorsqu'elle est alignée avec ladite séquence SEQ ID NO : 1.

5. Procédé selon la revendication 2, **caractérisé en ce que** ladite protéine TE de type FatA est choisie dans le groupe constitué parmi les séquences d'acides aminés SEQ ID NO : 1 à SEQ ID NO : 8.

6. Procédé selon l'une quelconque des revendications 1 à 5, **caractérisé en ce que** ladite plante est choisie parmi les plantes potagères, les plantes ornementales, les arbres fruitiers, le blé, le maïs, le riz, le cotonnier, le colza et le tournesol.

7. Utilisation d'un polynucléotide codant une protéine TE de type FatA telle que définie à l'une quelconque des revendications 1 à 5, pour induire chez une plante une tolérance à un stress hydrique.

## Patentansprüche

1. Verfahren zur Erhöhung der Wassermangeltoleranz einer Pflanze, **dadurch gekennzeichnet, dass** in der Pflanze ein Protein TE vom Typ FatA überexprimiert wird, das mindestens 60% Identität oder mindestens 70% Ähnlichkeit mit der Sequenz SEQ ID NO: 1 aufweist.

2. Verfahren nach Anspruch 1, **dadurch gekennzeichnet, dass** das Protein TE vom Typ FatA die konservierten Aminosäuren umfasst, die sich an den Positionen 70, 73-74, 76, 78, 80-82, 84-87, 89, 91-92, 94-97, 99-103, 105-114, 116, 118, 120, 123-132, 135-136, 138, 140-144, 249, 146-157, 159-168, 171, 174-180, 182-183, 187-189, 191-197, 200-202, 204-208, 210, 212, 214-217, 222-223, 226-235, 237-239, 241-242, 244, 246, 249, 251, 254-255, 257-283, 285, 287-291, 294-303, 305-306, 308-312, 323-325, 338-340, 342-343, 348, 350, 352-354, 356, 358-359 und 360 der Sequenz SEQ ID NO: 1 befinden, wenn es mit der Sequenz SEQ ID NO: 1 ausgerichtet ist.

3. Verfahren nach Anspruch 2, **dadurch gekennzeichnet, dass** das Protein TE vom Typ FatA von einer monokotyledonen Pflanze stammt und mindestens 79% Identität oder mindestens 79% Ähnlichkeit mit der Sequenz SEQ ID NO: 1 aufweist und die konservierten Aminosäuren umfasst, die sich an den Positionen 1-4, 8-10, 15, 19, 46, 51, 69-74, 76-87, 89-92, 94-116, 118-121, 123-133, 135-136, 138-168, 170-171, 174-180, 182-184, 187-189, 191-197, 200-202, 204-212, 214-219, 221-224, 226-239, 241-242, 244, 246, 249, 251-303, 305-312, 315, 317-318, 323-325, 330-331, 337-345, 348, 350, 352-359, 360-361 und 363 der Sequenz SEQ ID NO: 1 befinden, wenn es mit der Sequenz SEQ ID NO: 1 ausgerichtet ist.

4. Verfahren nach Anspruch 2, **dadurch gekennzeichnet, dass** das Protein TE vom Typ FatA von einer dikotyledonen Pflanze stammt und die konservierten Aminosäuren umfasst, die sich an den Positionen 24, 55, 61, 70, 73-74, 76, 78, 80-82, 84-87, 89, 91-92, 94-97, 99-103, 105-114, 116-118, 120, 122-132, 134-138, 140-144, 146-157, 159-168, 171, 173-183, 186-189, 191-202, 204-208, 210, 212, 214-217, 222-223, 226-235, 237-242, 244, 246-251, 254-255, 257-283, 285, 287-291, 294-303, 305-306, 308-312, 323-325, 328, 338-340, 342-343, 348, 350-354, 355 und 358-360 der Sequenz SEQ ID NO: 1 befinden, wenn es mit der Sequenz SEQ ID NO: 1 ausgerichtet ist.

5. Verfahren nach Anspruch 2, **dadurch gekennzeichnet, dass** das Protein TE vom Typ FatA in der Gruppe ausgewählt ist, die aus den Aminosäuresequenzen SEQ ID NO: 1 bis SEQ ID NO: 8 besteht.

6. Verfahren nach einem der Ansprüche 1 bis 5, **dadurch gekennzeichnet, dass** die Pflanze unter den Gemüsepflanzen, den Zierpflanzen, den Obstbäumen, dem Weizen, dem Mais, dem Reis, der Baumwollpflanze, dem Raps und der Sonnenblume ausgewählt ist.

7. Verwendung eines Polynukleotids, das ein Protein TE vom Typ FatA nach einem der Ansprüche 1 bis 5 codiert, um bei einer Pflanze eine Toleranz gegenüber Wasserknappheit zu bewirken.

## Claims

1. A method for increasing the tolerance of a plant to water deficit, **characterized in that** a FatA TE protein, having at least 60% identity or at least 70% similarity with the sequence SEQ ID No. 1, is overexpressed in said plant.

2. The method as claimed in claim 1, **characterized in that** said FatA TE protein comprises the conserved amino acids located at positions 70, 73-74, 76, 78, 80-82, 84-87, 89, 91-92, 94-97, 99-103, 105-114, 116, 118, 120, 123-132, 135-136, 138, 140-144, 249, 146-157, 159-168, 171, 174-180, 182-183, 187-189, 191-197, 200-202, 204-208, 210, 212, 214-217, 222-223, 226-235, 237-239, 241-242, 244, 246, 249, 251, 259-255, 257-283, 285, 287-291, 294-303, 305-306, 308-312, 323-325, 338-340, 342-343, 348, 350, 352-354, 356, 358-359 and 360 of said sequence SEQ ID No. 1 when it is aligned with said sequence SEQ ID No. 1.

3. The method as claimed in claim 2, **characterized in that** said FatA TE protein is derived from a monocotyledonous plant, and has at least 79% identity, or at least 79% similarity with the sequence SEQ ID NO: 1, and comprises the conserved amino acids located at positions 1-4, 8-10, 15, 19, 46, 51, 69-74, 76-87, 89-92, 94-116, 118-121, 123-133, 135-136, 138-168, 170-171, 174-180, 182-184, 187-189, 191-197, 200-202, 204-212, 214-219, 221-224, 226-239, 241-242, 244, 246, 249, 251-303, 305-312, 315, 317-318, 323-325, 330-331, 337-345, 348, 350, 352-359, 360-361 and 363 of said sequence SEQ ID NO: 1 when it aligned with said sequence SEQ ID NO: 1.

4. The method as claimed in claim 2, **characterized in that** said FatA TE protein is derived from a dicotyledonous plant, and comprises the conserved amino acids located at positions 24, 55, 61, 70, 73-74, 76, 78, 80-82, 84-87, 89, 91-92, 94-97, 99-103, 105-114, 116-118, 120, 122-132, 134-138, 140-144, 146-157, 159-168, 171, 173-183, 186-189, 191-202, 204-208, 210, 212, 214-217, 222-223, 226-235, 237-242, 244, 246-251, 254-255, 257-283, 285, 287-291, 294-303, 305-306, 308-312, 323-325, 328, 338-340, 342-343, 348, 350-354, 356 and 358-360 of said sequence SEQ ID NO: 1 when it aligned with said sequence SEQ ID NO: 1.

5. The method as claimed in claim 2, **characterized in that** said FatA TE protein is chosen from the group made up of the amino acid sequences SEQ ID NO: 1 to SEQ ID NO: 8.

6. The method as claimed in any one of claims 1 to 5, **characterized in that** said plant is chosen from edible plants, ornamental plants, fruit trees, wheat, corn, rice, cotton plant, rape and sunflower.

7. The use of a polynucleotide encoding a FatA TE protein as defined in any one of claims 1 to 5, for inducing water-stress tolerance in a plant.
